# DEMANDE DE BREVET EUROPEEN

(11) **EP 0 835 671 A1**
(43) Date de publication de la demande: **15.04.1998**
(21) Numéro de dépôt: 97402185.9
(22) Date de dépôt: 19.09.1997
(51) Int. Cl.: A61M 16/00

(54) **Appareil d'assistance respiratoire**

(30) Priorité: 11.10.1996 FR 9612424
(71) Demandeur: Boussignac, Georges, F-92160 Antony (FR); Labrune, Jean-Claude, F-92310 Sèvres (FR)
(72) Inventeur: Boussignac, Georges, F-92160 Antony (FR); Labrune, Jean-Claude, F-92310 Sèvres (FR)
(74) Mandataire: Bonnetat, Christian

(57) **Abrégé**

- La présente invention concerne un appareil d'assistance respiratoire.
- Selon l'invention, cet appareil comporte :
   . une pluralité de sorties (7.1 à 7.n), dont chacune d'elles peut assister un patient ;
   . une pluralité de liaisons (8.1 à 8.n) reliant respectivement une source de gaz respiratoire et l'une desdites sorties de l'appareil ;
   . une pluralité de liaisons (9.1 à 9.n) reliant respectivement des moyens de vanne commandés (5) alimentés en gaz respiratoire et l'une desdites sorties de l'appareil, de sorte que chacune desdites sorties de l'appareil reçoit une liaison de chacune des pluralités ; et
   . des moyens de réglage de débit (10.1 à 10.n) montés sur au moins certaines desdites liaisons.

## Description

La présente invention concerne les appareils d'assistance respiratoire.

On sait que de tels appareils comportent une source de gaz respiratoire, par exemple une bouteille de gaz sous pression, un générateur, etc ... et des moyens de vanne commandés, alimentés par ladite source de gaz respiratoire et engendrant à leurs sorties des impulsions de gaz respiratoire dont ils contrôlent la période et la durée.

De tels appareils connus sont individuels et permettent de n'assister qu'un seul patient.

Par le contrôle des moyens de vanne commandés, il est possible, en fonction du patient, de régler dans le temps et successivement la durée de l'inspiration et la durée de l'expiration, tout en respectant les temps morts souhaités et réglés par un opérateur.

Par suite, un tel appareil individuel permet d'optimiser l'assistance respiratoire au patient qui y est relié. Toutefois, dans le cas de catastrophes ou d'accidents graves entraînant de nombreuses victimes ayant besoin simultanément d'une assistance respiratoire, il est indispensable de prévoir autant d'appareils respiratoires que de victimes.

Ceci n'est généralement pas possible, de sorte que certaines victimes ne peuvent pas être assistées avant leur arrivée dans un centre hospitalier. Cela est bien entendu préjudiciable à leur santé, et peut même leur être fatal, alors qu'elles auraient pu être sauvées si on avait pu leur apporter une assistance respiratoire même imparfaite, mais rapide.

On remarquera toutefois que le document US-A-4 495 946, qui décrit un appareil respiratoire impulsionnel du type rappelé ci-dessus, mentionne que ledit appareil peut être utilisé pour plusieurs patients simultanément à partir d'une unique source d'oxygène commune et grâce à une seule vanne commandée. Il est bien évident que, dans ce cas, le même rythme respiratoire est imposé à tous les malades, ce qui peut être nuisible et même fatal pour certains d'entre eux, sans possibilité d'autre réglage que le débit d'oxygène pulsé.

La présente invention a pour objet de remédier aux inconvénients précités et elle concerne un appareil susceptible d'apporter une assistance respiratoire rapide et efficace, simultanément à une pluralité de patients.

A cette fin, selon l'invention, l'appareil d'assistance respiratoire comportant une source de gaz respiratoire et des moyens de vanne commandés, alimentés par ladite source de gaz respiratoire et engendrant à leurs sorties des impulsions de gaz respiratoire dont ils contrôlent la période et la durée, ledit appareil comportant une pluralité de sorties, dont chacune d'elles peut assister un patient et une première pluralité de liaisons reliant respectivement lesdits moyens de vanne commandés et l'une desdites sorties de l'appareil, est remarquable en ce qu'il comporte :
- une seconde pluralité de liaisons reliant respectivement ladite source de gaz respiratoire et l'une desdites sorties de l'appareil, de sorte que chacune desdites sorties de l'appareil reçoit une liaison de ladite première pluralité et une liaison de ladite seconde pluralité ; et
- des moyens de réglage de débit montés sur au moins certaines des liaisons desdites première et seconde pluralités.

Ainsi, chaque patient reçoit, en plus d'impulsions de gaz respiratoire, un débit de gaz respiratoire à pression constante qui permet de maintenir une pression positive permanente dans les poumons des patients. Outre le fait que cette pression positive permanente atténue les effets de l'éventuelle unicité de rythme respiratoire, elle permet :
- de maintenir ouverts les alvéoles pulmonaires des patients ;
- d'éliminer le gaz carbonique hors des poumons ; et
- d'améliorer l'oxygénisation des patients.

La qualité de l'assistance respiratoire délivrée par l'appareil conforme à la présente invention est donc particulièrement bonne.

Lesdits moyens de réglage de débit peuvent comporter, pour chaque sortie dudit appareil, un premier et un second robinets, respectivement montés sur la liaison de la première pluralité et sur la liaison de la seconde pluralité arrivant à ladite sortie de l'appareil respiratoire.

Grâce à une telle disposition, il est donc possible de commander aussi bien le débit du gaz respiratoire à pression constante de ladite source, que le débit desdites impulsions de gaz respiratoire.

En variante, chacun desdits moyens de réglage de débit associé à une sortie dudit appareil peut être constitué par une vanne, dite "proportionnelle", dont l'entrée est reliée, à la fois, à la liaison de la première pluralité et à la liaison de la seconde pluralité arrivant à ladite sortie de l'appareil. En effet, on sait qu'une telle vanne s'ouvre proportionnellement au débit qu'elle reçoit, de sorte qu'elle est particulièrement appropriée à additionner lesdites impulsions de gaz respiratoire audit débit de gaz respiratoire à pression constante.

De préférence, lesdits moyens de vanne commandés sont réglables pour permettre d'ajuster la période et la durée desdites impulsions de gaz respiratoire. Ainsi, il est possible de régler l'appareil conforme à l'invention à une période et une durée d'impulsions convenant au moins approximativement à chacun des patients assistés.

Dans un premier mode de réalisation particulièrement simple de l'appareil conforme à la présente invention, lesdits moyens de vanne commandés sont constitués par une vanne commandée unique, commune à toutes les liaisons de ladite première pluralité. L'appareil conforme à la présente invention dispense donc alors à tous les patients des impulsions de gaz de période et de durée identiques, l'individualisation de l'assistance respiratoire s'effectuant par commande des débits de gaz continus et impulsionnels, grâce auxdits robinets.

On voit donc, dans ce premier mode de réalisation, que l'appareil d'assistance respiratoire conforme à la présente invention n'est pas optimisé pour chacun des patients assistés, mais il permet d'apporter à une pluralité de patients une assistance respiratoire de qualité, quoique de compromis, maintenant lesdits patients en vie jusqu'à ce que ceux-ci puissent bénéficier d'une assistance respiratoire individualisée et optimisée. De plus, sa mise en oeuvre est particulièrement simple puisque seuls les robinets de commande des débits continus et impulsionnels doivent être actionnés, de sorte qu'il peut être servi par du personnel relativement peu qualifié.

Dans un deuxième mode de réalisation, plus sécuritaire et légèrement plus complet, lesdits moyens de vanne commandés sont constitués par une pluralité de vannes commandées individuelles, dont chacune d'elles est montée sur une desdites liaisons de la première pluralité.

On voit qu'ainsi la période et la durée des impulsions de gaz respiratoire peuvent être adaptées aux besoins de chacun des patients.

L'appareil conforme à la présente invention peut comporter un réservoir d'accumulation de gaz respiratoire disposé entre la source de gaz respiratoire et lesdits moyens de vanne commandés.

De même, ledit appareil peut comporter un autre réservoir d'accumulation de gaz respiratoire disposé entre ladite source de gaz respiratoire et lesdites liaisons de la seconde pluralité.

Ainsi, lesdits réservoirs constituent les cavités tampon.

Avantageusement, l'appareil conforme à la présente invention comporte une pluralité d'organes individuels d'adaptation aux patients, tels que sondes respiratoires, masques respiratoires ou analogues, chacun alimenté en gaz par l'une desdites sorties de l'appareil. Dans le cas de sondes, celles-ci sont avantageusement du type décrit dans le brevet européen EP-A-0 366 524.

Par ailleurs, par sécurité, l'appareil comporte un système de mesure de pression relié respectivement auxdits organes individuels d'adaptation. Il est ainsi possible de vérifier que le système respiratoire d'aucun des patients ne subit une pression excessive.

On remarquera par ailleurs que le document EP-A-0 097 060, décrit un appareil d'assistance respiratoire à deux sorties sur lesquelles apparaissent, soit un flux impulsionnel, soit un flux continu de gaz respiratoire.

Les figures du dessin annexé feront bien comprendre comment l'invention peut être réalisée. Sur ces figures, des références identiques désignent des éléments semblables.

Ces figures illustrent, schématiquement et respectivement, deux exemples de réalisation de l'appareil respiratoire conforme à la présente invention.

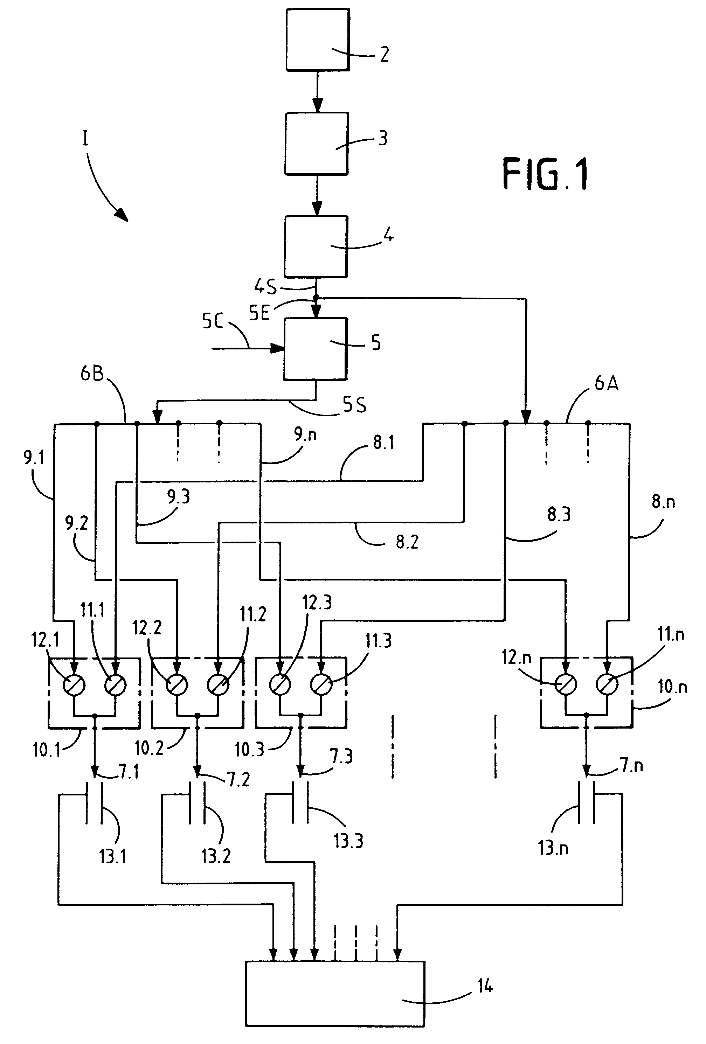

L'appareil respiratoire I, montré par la figure 1, comporte une source de gaz respiratoire 2, par exemple une bouteille de gaz sous pression ou un générateur de gaz, éventuellement associée à un détendeur 3. La sortie du détendeur 3 est reliée à un éventuel réservoir 4.

La sortie 45 du réservoir 4 est reliée, d'une part, à l'entrée 5E d'une vanne commandée 5 et, d'autre part, à une première conduite de distribution 6A. La sortie 5S de la vanne commandée 5 est par ailleurs reliée à une seconde conduite de distribution 6B.

La vanne commandée 5 comporte une entrée de commande 5C, reliée à un dispositif de commande (non représenté), de sorte que l'ensemble de la source 2 et du détendeur 3 peut adresser à la conduite de distribution 6B des impulsions de gaz respiratoire. Le dispositif de commande contrôle la vanne 5 pour que la période et la durée des impulsions de gaz respiratoire puissent être réglables.

L'appareil d'assistance respiratoire 1 comporte une pluralité de sorties 7.1, 7.2, 7.3, ..., 7.n dont chacune d'elles est reliée par des liaisons respectives 8.1, 8.2, 8.3, ..., 8.n et 9.1, 9.2, 9.3, ..., 9.n, à la fois à la conduite de distribution 6A et à la conduite de distribution 6B. Par ailleurs, des moyens de réglage de débit 10.1, 10.2, 10.3, ..., 10.n sont montés sur les liaisons 8.1 à 8.n et 9.1 à 9.n, afin de régler les échanges gazeux entre les conduites de distribution 6A et 6B et les sorties 7.1 à 7.n. Chacun desdits moyens de réglage de débit 10.1 à 10.n peut être constitué par une vanne proportionnelle. Cependant, en variante, comme cela est montré par la figure 1, chaque moyen de réglage de débit 10.i (avec i = 1, 2, 3, ..., n) peut comporter un robinet 11.i monté sur la liaison 8.i correspondante et un robinet 12.i monté sur la liaison 9.i correspondante.

Chacune des sorties 7.1 à 7.n est reliée à un organe individuel d'adaptation à un patient 13.1, 13.2, 13.3, ..., 13.n, tel que sonde, masque ou analogue. Ainsi, grâce à l'entrée de commande 5C, il est possible de choisir, pour l'ensemble des patients reliés aux organes 13.i, la période et la durée des impulsions de gaz respiratoire convenant à leur ensemble. De plus, les patients subissent chacun une ventilation continue grâce au courant de gaz respiratoire continu transitant par la conduite de distribution 6A. L'adaptation de l'assistance respiratoire au cas de chacun des patients est assurée par le contrôle du débit par la commande des robinets 10.i et 12.i associés.

Par ailleurs, un dispositif manométrique 14, relié individuellement à chacun des organes 13.1 à 13.n permet de contrôler la pression dans chacune de ceux-ci.

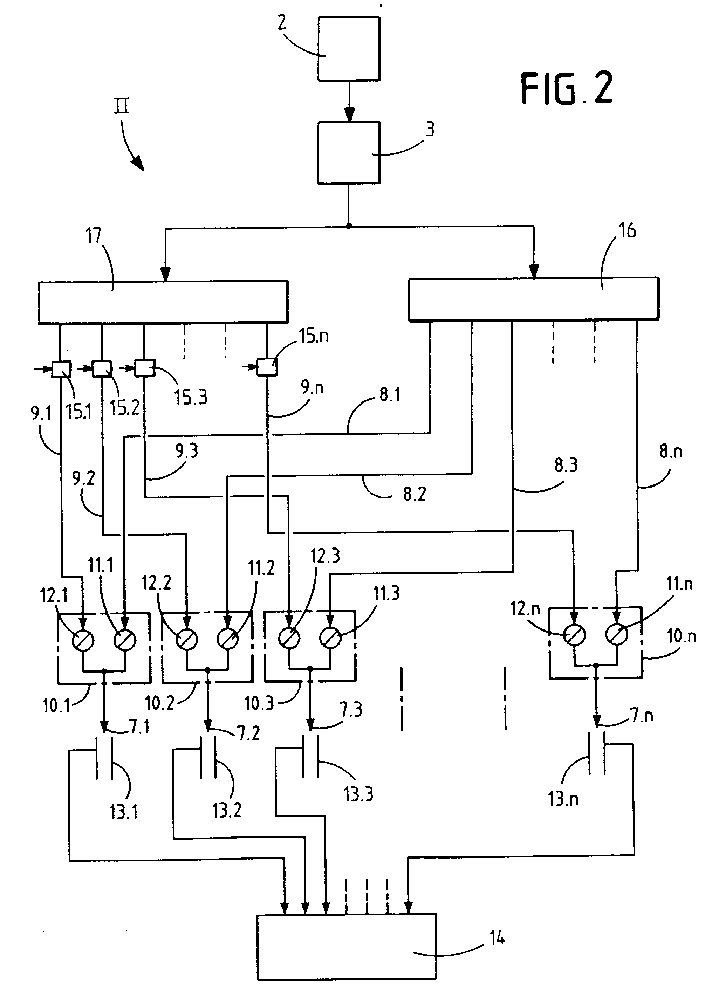

Dans l'exemple de réalisation II de l'appareil de l'invention, représenté sur la figure 2, on retrouve la source de gaz respiratoire 2, le détendeur 3, les sorties 7.1 à 7.n, les liaisons 8.1 à 8.n, les liaisons 9.1 à 9.n, les moyens de réglage de débit 10.1 à 10.n avec leurs robinets 11.1 à 11.n et 12.1 à 12.n, les organes d'adaptation 13.1 à 13.n et le dispositif manométrique 14. Cependant, dans cette variante de réalisation, la vanne commandée 5, commune à toutes les lignes 9.1 à 9.n, a été remplacée par une pluralité de vannes commandées individuelles 15.1 à 15.n, chacune montée sur une liaison 8.1 à 8.n. Les vannes commandées 15.1 à 15.n comportent chacune une entrée de commande individuelle, de façon à pouvoir être commandées individuellement par un dispositif de commande (non représenté). Ainsi, l'appareil peut délivrer à chacune de ses sorties 7.1 à 7.n des impulsions de gaz respiratoire dont la période et la durée sont optimales pour le patient relié à l'organe 13.1 à 13.n correspondant.

Par ailleurs, dans la variante de réalisation II de la figure 2, le réservoir 4 est remplacé par deux réservoirs 16 et 17, en parallèle, faisant office respectivement de conduites de distribution 6A et 6B, toutes les conduites 8.1 à 8.n étant reliées au réservoir 16 et toutes les conduites 9.1 à 9.n provenant du réservoir 17.

## Revendications

1. Appareil d'assistance respiratoire comportant une source de gaz respiratoire (2, 3) et des moyens de vanne commandés (5, 15.1, ..., 15.n), alimentés par ladite source de gaz respiratoire et engendrant à leurs sorties des impulsions de gaz respiratoire dont ils contrôlent la période et la durée, ledit appareil comportant une pluralité de sorties (7.1 à 7.n), dont chacune d'elles peut assister un patient et une première pluralité de liaisons (9.1 à 9.n) reliant respectivement lesdits moyens de vanne commandés (5, 15.1 à 15.n) et l'une desdites sorties de l'appareil,
caractérisé en ce qu'il comporte :
- une seconde pluralité de liaisons (8.1 à 8.n) reliant respectivement ladite source de gaz respiratoire et l'une desdites sorties de l'appareil, de sorte que chacune desdites sorties de l'appareil reçoit une liaison de ladite première pluralité et une liaison de ladite seconde pluralité ; et
- des moyens de réglage de débit (10.1 à 10.n) montés sur au moins certaines des liaisons desdites première et seconde pluralités.

2. Appareil d'assistance respiratoire selon la revendication 1,
caractérisé en ce que lesdits moyens de réglage de débit (10.1 à 10.n) comportent, pour chaque sortie dudit appareil, un premier (12.1 à 12.n) et un second (11.1 à 11.n) robinets, respectivement montés sur la liaison (9.1 à 9.n) de la première pluralité et sur la liaison (8.1 à 8.n) de la seconde pluralité arrivant à ladite sortie (7.1 à 7.n) de l'appareil respiratoire.

3. Appareil d'assistance respiratoire selon la revendication 1,
caractérisé en ce que lesdits moyens de réglage de débit (10.1 à 10.n) comportent, pour chaque sortie dudit appareil, une vanne proportionnelle, dont l'entrée est reliée, à la fois, à la liaison de ladite première pluralité et à la liaison de ladite seconde pluralité arrivant à ladite sortie (7.1 à 7.n) de l'appareil respiratoire.

4. Appareil d'assistance respiratoire selon l'une des revendications 1 à 3,
caractérisé en ce que lesdits moyens de vanne commandés (5, 15.1 à 15.n) sont réglables pour permettre de régler ladite période et ladite durée des impulsions de gaz à une valeur désirée.

5. Appareil d'assistance respiratoire selon l'une des revendications 1 à 4,
caractérisé en ce que lesdits moyens de vanne commandés sont constitués par une vanne commandée unique (5), commune à toutes lesdites liaisons (9.1 à 9.n) de la première pluralité.

6. Appareil d'assistance respiratoire selon l'une des revendications 1 à 4,
caractérisé en ce que lesdits moyens de vanne commandés sont constitués par une pluralité de vannes commandées individuelles (15.1 à 15.n), dont chacune d'elles est montée sur une desdites liaisons (9.1 à 9.n) de la première pluralité.

7. Appareil d'assistance respiratoire selon l'une des revendications 1 à 6,
caractérisé en ce qu'il comporte un réservoir (4, 17) d'accumulation de gaz respiratoire disposé entre ladite source de gaz respiratoire (2, 3) et lesdits moyens de vanne commandés (5, 15.1 à 15.n).

8. Appareil d'assistance respiratoire selon l'une des revendications 1 à 7,
caractérisé en ce qu'il comporte un réservoir d'accumulation de gaz respiratoire (4, 16) relié, d'une part, à ladite source de gaz respiratoire (2, 3) et, d'autre part, auxdites liaisons (8.1 à 8.n) de la seconde pluralité.

9. Appareil d'assistance respiratoire selon l'une des revendications 1 à 8,
caractérisé en ce qu'il comporte une pluralité d'organes individuels d'adaptation aux patients (13.1 à 13.n), chacun alimenté en gaz respiratoire par l'une desdites sorties (7.1 à 7.n) dudit appareil.

10. Appareil d'assistance respiratoire selon la revendication 9,
caractérisé en ce qu'il comporte un système de mesure de pression (14) relié respectivement auxdits organes individuels d'adaptation (13.1 à 13.n).
